# EUROPEAN PATENT SPECIFICATION

(11) **EP 1 272 049 B1**
(45) Date of publication and mention of the grant of the patent: **04.11.2009**
(21) Application number: 01932031.6
(22) Date of filing: 12.04.2001
(51) Int. Cl.: A23J 1/08, C11B 1/00, C11B 7/00, A23D 9/013, A23J 7/00

(54) **METHOD FOR THE FRACTIONATION OF OIL AND POLAR LIPID-CONTAINING NATIVE RAW MATERIALS USING ALCOHOL AND CENTRIFUGATION**
VERFAHREN ZUR FRAKTIONIERUNG VON ÖL- UND POLAR LIPID-ENTHALTENDEN ROHSTOFFEN UNTER VERWENDUNG VON ALKOHOL UND ZENTRIFUGATION
PROCEDE POUR FRACTIONNER DES MATIERES PREMIERES NATIVES CONTENANT DE L'HUILE ET DES LIPIDES POLAIRES AU MOYEN DE SOLVANT ORGANIQUE HYDROSOLUBLE ET DE CENTRIFUGATION

(30) Priority: 12.04.2000 DE 10018213; 23.02.2001 US 271209 P
(43) Date of publication of application: 08.01.2003
(73) Proprietor: GEA Westfalia Separator GmbH, 59302 Oelde (DE)
(72) Inventor: HRUSCHKA, Steffen, M., D-59302 Oelde (DE); KIRCHNER, Stefan, D-33334 Gütersloh (DE); RASSENHOVEL, Jürgen, D-59302 Oelde (DE); WITT, Willi, D-49545 Tecklenburg (DE); BEST, Bernd, D-64546 Mörfelden (DE)
(74) Representative: Specht, Peter
(86) International application number: PCT/IB2001/000963
(87) International publication number: WO 2001/076385

(56) References cited:
- WO-A-97/27274
- CA-A- 1 335 054
- US-A- 4 157 404
- US-A- 5 883 273
- US-A- 5 917 068

## Description

### FIELD OF THE INVENTION

The present invention relates to a process for fractionation of an oil-, polar lipid, and protein-containing mixture obtained from microbes.

### BACKGROUND OF THE INVENTION

Examples of polar lipids include phospholipids (e.g. phosphatidyl choline, phosphatidyl ethanolamine, phosphatidyl inositol, phosphatidyl serine, phosphatidylglycerol, diphosphatidylglycerols), cephalins, sphingolipids (sphingomyelins and glycosphingolipids), and glycoglycerolipids. Phospholipids are composed of the following major structural units: fatty acids, glycerol, phosphoric acid, amino alcohols, and carbohydrates. They are generally considered to be structural lipids, playing important roles in the structure of the membranes of plants, microbes and animals. Because of their chemical structure, polar lipids exhibit a bipolar nature, exhibiting solubility or partial solubility in both polar and non-polar solvents. The term polar lipid within the present description is not limited to natural polar lipids but also includes chemically modified polar lipids. Although the term oil has various meanings, as used herein, it will refer to the triacylglycerol fraction.

One of the important characteristics of polar lipids, and especially phospholipids, is that they commonly contain polyunsaturated fatty acids (PUFAs: fatty acids with 2 or more unsaturated bonds). In many plant, microbial and animal systems, they are especially enriched in the highly unsaturated fatty acids (HUFAs: fatty acids with 4 or more unsaturated bonds) of the omega-3 and omega-6 series. Although these highly unsaturated fatty acids are considered unstable in triacylglycerol form, they exhibit enhanced stability when incorporated in phospholipids.

The primary sources of commercial PUFA-rich phospholipids are soybeans and canola seeds. These biomaterials do not contain any appreciable amounts of HUFAs unless they have been genetically modified. The phospholipids (commonly called lecithins) are routinely recovered from these oilseeds as a byproduct of the vegetable oil extraction process. For example, in the production of soybean or canola oil, the beans (seeds) are first heat-treated and then crushed, ground, and/or flaked, followed by extraction with a non-polar solvent such as hexane. Hexane removes the triacylglycerol-rich fraction from the seeds together with a varying amount of polar lipids (lecithins). The extracted oil is then degummed (lecithin removal) either physically or chemically as a part of the normal oil refming process and the precipitated lecithins recovered. One disadvantage of this process is the use of the non-polar solvents such as hexane presents toxicity and flammability problems that must be dealt with.

The crude lecithin extracted in the "de-gumming" process can contain up to about 33% oil (triacylglycerols). One preferred method for separating this oil from the crude lecithin is by extraction with acetone. The oil (triacylglycerols) is soluble in acetone and the lecithin is not. The acetone solution is separated from the precipitate (lecithin) by centrifugation and the precipitate dried under first a fluidized bed drier and then a vacuum drying oven to recover the residual acetone as the product is dried. Drying temperatures of 50-70°C are commonly used. The resulting dried lecithins contain approximately 2-4% by weight of oil (triacylglycerols). Process temperatures above 70°C can lead to thermal decomposition of the phospholipids. However, even at temperatures below 70°C the presence of acetone leads to the formation of products that can impair the organoleptic quality of the phospholipids. These by-products can impart musty odors to the product and also a pungent aftertaste.

To avoid use of non-polar solvents such as hexane and avoid the negative side effects of an acetone-based process, numerous processes have also been proposed involving the use of supercritical fluids, especially supercritical CO₂. For example, U.S. Patent No. 4,367,178 discloses the use of supercritical CO₂ to partially purify crude soy lecithin preparation by removing the oil from the preparation. German Patent Nos. DE-A 30 11 185 and DE-A 32 29 041 disclose methods for de-oiling crude lecithin with supercritical CO₂ and ethane respectively. Other supercritical processes have been proposed which include adding small amounts of hydrocarbons such as propane to the supercritical CO₂ to act as entraining agents. However, supercritical fluid extraction systems are very capital expensive and cannot be operated continuously. Further, extraction times are long and the biomaterials must be dried before extraction, and this increases the difficulties of stabilizing the resulting dry product with antioxidants. All of these factors make the supercritical process one of the most expensive options for extracting and recovering polar-lipid material or mixtures of these materials. As a result, alternative processes using extraction with liquid hydrocarbons at lower pressures have been described. For example U.S. Patent No. 2,548,434 describes a method for de-oiling oilseed materials and recovering crude lecithin using a liquid hydrocarbon at lower pressures (35-45 bars) but elevated temperatures (79° to 93°C). U.S. Patent No. 5,597,602 describes a similar process that operates at even lower pressures and temperatures. However, even with these improvements supercritical fluid extraction remains very expensive and is not currently used to produce phospholipids for food use on a large commercial scale.

The primary commercial source of HUFA-rich polar lipids is egg yolk. Two primary methods are used for the recovery of egg phospholipids on an industrial scale. Both require the drying of the egg yolk before extraction. In the first process the dried egg yolk powder is extracted first with acetone to remove the triacylglycerols. This is then followed by an extraction with pure alcohol to recover the phospholipids. In the second process, pure alcohol is used to extract an oil/lecithin fraction from the dried egg yolk. The oil/lecithin phase is then extracted with acetone to remove the triacylglycerols, leaving behind a lecithin fraction. Both of these methods require the use of acetone, which has the disadvantages discussed above.

Canadian Patent No. 1,335,054 describes a process for extracting fresh liquid egg yolk into protein, oil and lecithin fractions by the use of ethanol, elevated temperatures, filtration and low temperature crystallization with further filtration. The purity of the lecithin product is not disclosed. However one skilled in the art would expect that the lecithin fraction produced by this process would not be very pure. There would still be very significant amounts of oil associated with the lecithin because the chilling process would primarily remove the triglycerides containing saturated fatty acids. Those containing some unsaturated fatty acids would remain more soluble at low temperatures. Additionally, the filtration and the chilling/filtration processes employed in this method would be labor intensive and difficult to turn into a continuous process. In light of the current state of the art, there remains a need for an improved extraction technology for food-grade polar lipid products that is less expensive to operate and which protects the overall quality of the HUFAs in the polar lipid products.

US-A-5 883 273 discloses a process for separating phospholipids and tryglycerids from egg yolk comprising the steps of: adding the lower alcohol methanol to the egg yolk, separating the insoluble protein from a methanolic solution of lipids, adding water, and centrifuging in order to obtain a tryglyceride phase and a phospholipid phase.

### SUMMARY OF THE INVENTION

In accordance with the present invention, an improved process is provided for recovering polar lipids from native biomaterials, i.e. microbes, which does not involve the disadvantages of the prior art.

The invention provides the processes of claims 1 and 16. Preferred embodiments are the subject matter of the subclaims.

The process preferably includes the steps of adding a high concentration water-soluble organic solvent to the oil-, polar lipid-, and protein-containing mixture, and separating protein from the mixture to form a protein-rich fraction and a polar lipid/oil-rich fraction. As used herein, the term "high concentration water-soluble organic solvent" will mean greater than 68 percent organic solvent, preferably greater than 80 percent organic solvent, more preferably greater than 90 percent, more preferably from about 80 percent to about 95 percent organic solvent.
Preferably, the process steps are conducted under oxygen-reduced atmospheres that can include use of inert or non-reactive gases (e.g. nitrogen, carbon dioxide, argon, etc), use of solvent vapors, use of a partial or full vacuum, or any combination of the above.

### BRIEF DESCRIPTION OF THE FIGURES

The present invention may be more readily understood by reference to the following figures, wherein
FIG. 1 is a graphical representation of the solubility of phospholipids, a form of polar lipids, as a function of alcohol concentration.
FIG. 2 is a graphical representation of a phospholipid extraction process (as an example of a polar lipid extraction process) based on a high concentration of alcohol.

### DETAILED DESCRIPTION OF THE INVENTION

Because of their bipolar nature, polar lipids (including phospholipids) are of significant commercial interest as wetting and emulsifying agents. These properties may also help make HUFAs in the phospholipids more bioavailable, in addition to enhancing their stability. These properties make phospholipids ideal forms of ingredients for use in nutritional supplements, food, infant formula and pharmaceutical applications.

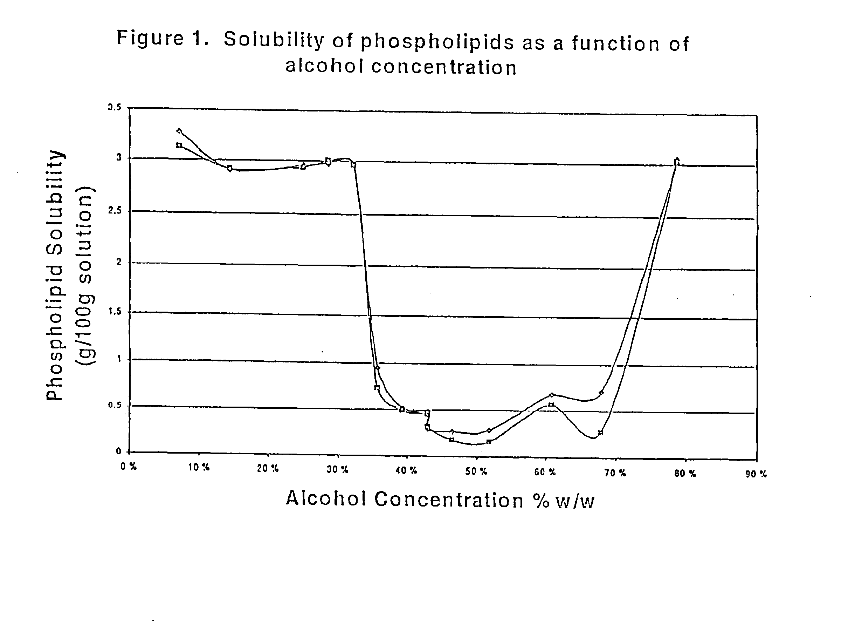

We have unexpectedly found that polar lipids are soluble not only in high water-soluble organic solvent concentrations (e.g., at water-soluble organic solvent concentrations greater than about 68% w/w) but also in low water-soluble organic solvent concentrations (less than about 35% water-soluble organic solvent w/w) (FIG. 1). As used herein, water-soluble organic solvent concentration means the weight percentage of water-soluble organic solvent in an aqueous solution. The aqueous solution includes added water and water present in the materials. For the purpose of this invention, phospholipids are described as "soluble" if they do not settle or separate from the continuous phase (sometimes also called supernatant or light phase) when subjected to centrifugation by equipment described in this invention. In the water-soluble organic solvent concentration range from about 35% w/w to about 68% w/w water-soluble organic solvent, polar lipids exhibit significantly lower solubility. The present invention exploits this property of polar lipids (enhanced solubility/dispersibility at low water-soluble organic solvent concentrations), which can then be exploited in several ways (along with the high solubility of phospholipids in high water-soluble organic solvent concentrations) to develop processes for inexpensively extracting and recovering polar lipids, and especially phospholipids, from native biomaterials.

Native biomaterials that are rich in HUFA-containing polar lipids include fish, crustaceans, microbes, eggs, brain tissue, milk, meat and plant material including oilseeds. As used herein, the terms fish, crustaceans, microbes, eggs, brain tissue, milk, meat and plant material including oilseeds will include genetically modified versions thereof. The content of phospholipids in these materials is generally low, usually ranging from 0.1% to about 4% by wet weight. As a result large amounts of raw materials need to be processed to recover these phospholipids. Because of the high costs of prior extraction techniques, phospholipids and especially HUFA-enriched phospholipids were very expensive and therefore restricted to use in the infant formula, pharmaceutical and cosmetic industries. One of the advantages of the present invention is that it provides for the extraction of polar lipids, and in particular phospholipids, in a cost-effective manner.

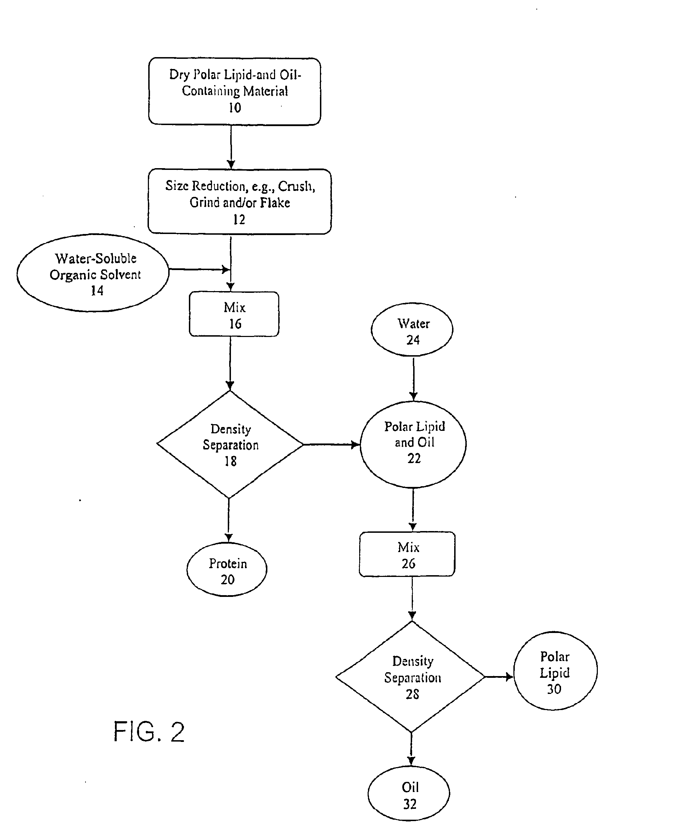

A polar lipid recovery process utilizing high concentrations of water-soluble organic solvent in a polar lipid/oil concentration step followed by the use of low water-soluble organic solvent concentrations in a step recovering the polar lipids from the oil phase is outlined in FIG. 2. Liquid egg yolk is used as the polar-lipid rich biomaterial in this example. It is understood, however, that other polar lipid-containing biomaterials (e.g. fish, crustaceans, microbes, brain tissue, milk, meat and plant material including oilseeds) could also be processed in a similar manner with minor modification to the process.

In the first step of the process 12, the material is dried, if necessary. For a more efficient recovery of the protein, the material is optionally subjected to size reduction 14. A water-soluble organic solvent (e.g., alcohol) is added 14. The concentration of water-soluble organic solvent in the water-soluble organic solvent/water solution is at least about 68% w/w, preferably at least about 80% w/w, preferably at least about 90% w/w, more preferably from about 80 to about 95% w/w, more preferably from about 85 to about 95% w/w, and more preferably from about 90 to about 95% w/w. The more moisture that is present in the material, the greater the amount and/or the higher the concentration of the water-soluble organic solvent that will be needed to achieve the desired concentration when mixed with the material. In other words, if the material is relatively dry, less water-soluble organic solvent and/or lower concentration water-soluble organic solvent can be employed. On the other hand, if the material is relatively wet, more water-soluble organic solvent and/or higher concentration water-soluble organic solvent must be employed.

The denatured protein 20 is then separated by density separation 18. Since proteins are not soluble in high concentrations of water-soluble organic solvent, they precipitate (while the polar lipids and oil dissolves in the high concentration water-soluble organic solvent) and the precipitated proteins 20 are separated from the polar lipid/oil-enriched fraction 22 by density separation 18, e.g., using gravity or centrifugal force. Using egg yolk as an example, this results in two fractions being recovered: (1) a fraction with approximately 60-95% oil (as % dry weight) and about 5-40% dry weight as polar lipids; and (2) a protein fraction, preferably with more than 90% of the protein of the egg yolk.

If it is desired to separate the polar lipid from the oil, the oil/polar lipid fraction 22 is mixed 26 with water 24 to a final concentration of water-soluble organic solvent in water of from about 25 to about 30% w/w. A less desirable alternative would be to dry the oil/polar lipid fraction 22 and then add a water-soluble organic solvent, and water as necessary, to achieve the desired concentration of water-soluble organic solvent. The polar lipid is then separated from the oil by means of density separation 28. A polar lipid-enriched fraction 30 and an oil-enriched fraction 32 are formed. Further processing can be performed on the polar lipid-enriched fraction 30 and/or oil-enriched fraction 32 as desired or necessary. For example, counter-current washing/centrifugation or cross-current washing/separation of the oil and polar lipid products can be employed to improve the purity of the products and economics of the overall process.

In an alternative embodiment, the drying step can be eliminated. For example, instead of drying a material such as eggs, wet eggs can be used. The process is similar to that described above, however, the drying step is eliminated. As a result, a larger amount and/or higher concentration of water-soluble organic solvent is employed to precipitate the protein.

Because of the simplicity of the equipment required in the process, the entire process can very easily be conducted under a reduced-oxygen atmosphere (e.g., nitrogen, a preferred embodiment of the process), further protecting any HUFAs in the polar lipids from oxidation. For example, a gas tight decanter can be used to separate protein from the mixture. A suitable decanter is model CA 226-28 Gas Tight available from Westfalia Separator Industry GmbH of Oelde Germany, which is capable of continuous separation of protein from suspensions with high protein solids content in a centrifugal field. A gas tight separator useful for separating polar lipids from oil is model SC 6-06-576 Gas Tight available from Westfalia Separator Industry GmbH of Oelde Germany, which is capable of continuous separation of polar lipids from oil in a centrifugal field.

The concentration of water-soluble organic solvent in the protein removal step is preferably greater than about 68% w/w, more preferably greater than about 70% w/w, more preferably greater than about 80% w/w, more preferably greater than about 90% w/w. In principle, it is believed that the higher the water-soluble organic solvent concentration, the stronger the protein contraction, but the more nonpolar the aqueous/water-soluble organic solvent phase, more polar lipids may be dissolved in the oil phase. The appropriate concentration and temperature must therefore be found, for example, by conducting a few preliminary experiments (centrifuge tests), for each raw material.

## Claims

1. A process for fractionation of an oil-, polar lipid, and protein-containing mixture obtained from microbes, comprising the steps:
a) adding a water-soluble organic solvent to said mixture and separating protein from said mixture to form a protein-rich fraction and a polar-lipid/oil-rich fraction,
b) reducing the concentration of water-soluble organic solvent in said polar-lipd/oil-rich fraction, and
c) subjecting the water/water-soluble organic solvent and polar lipid/oil-rich fraction to density separation to form a polar-lipid-rich fraction and an oil-rich fraction,
d) said polar lipid/oil-rich fraction formed in step (a) comprising from 5% to 40% by weight polar lipid and from 60% to 95% by weight oil, and
e) said protein-rich fraction formed in step (a) comprising from 80% to 95% by weight protein on a dry basis, and
f) said water-soluble organic solvent in step (a) being present in a water-soluble organic solvent/water mixture in which said water-soluble organic solvent/water mixture comprises at least 68% by weight water-soluble organic solvent;
g) wherein said water-soluble organic solvent in step (c) is present in a water-soluble organic solvent/water mixture in which said said water-soluble organic solvent/water mixture comprises from 25% to 30% by weight water-soluble organic solvent.

2. The process of Claim 1, wherein the separation of protein of step (a) comprises the steps:
(a) adding water-soluble organic solvent to said oil-, polar lipid-, and protein-containing mixture to obtain a water-soluble organic solvent concentration of a least 80% w/w; and
(b) separating by density separation the resulting mixture into a protein-rich fraction and a polar lipid/oil-rich fraction.

3. The process of Claim 1, wherein water-soluble organic solvent is recovered from the protein-rich fraction and the polar lipid/oil-rich fraction after the density separation.

4. The process of Claim 1, wherein said oil-, polar lipid-, and protein-containing mixture further comprises cholesterol and a substantial amount of said cholesterol reports to said oil-rich fraction pursuant to the separation of step (c).

5. The process of Claim 1, wherein said water-soluble organic solvent in step (a) is present in a water-soluble organic solvent/water mixture in which said said water-soluble organic solvent/water mixture comprises from 80% to 95% by weight water-soluble organic solvent.

6. The process of Claim 1, wherein said water-soluble organic solvent is recovered by countercurrent washing, evaporation or drying.

7. The process of Claim 1, wherein said polar lipid-rich fraction is dried to recover water-soluble organic solvent, washed with an water-soluble organic solvent/water mixture comprising greater than 80% by weight water-soluble organic solvent in order to precipitate residual protein and further dried to recover the water-soluble organic solvent.

8. The process of Claim 7, wherein the addition of said water-soluble organic solvent results in the precipitation of at least some of said protein, which is recovered by density separation.

9. The process as claimed in Claims 1- 8, wherein said water-soluble organic solvent comprises a polar solvent.

10. The process as claimed in Claims 1-9, wherein said water-soluble organic solvent comprises an alcohol.

11. The process as claimed in Claims 1-10, wherein said water-soluble organic solvent comprises a C₁-C₈ alcohol.

12. The process as claimed in Claims 1-11, wherein said water-soluble organic solvent comprises isopropanol, ethanol or mixtures thereof.

13. The process as claimed in Claims 1-12, wherein the pH during processing is from pH 4 to pH 10.

14. The process as claimed in Claims 1-13, wherein at least 60% of the polar lipids originally present in the mixture are recovered in a polar lipid-rich fraction.

15. The process as claimed in Claims 1-14, wherein at least 80% of the polar lipids originally present in the mixture are recovered in a polar lipid-rich fraction.

16. A process for recovering polar lipid from a polar lipid-containing mixture obtained from microbes, employing the use of a water-soluble organic solvent, wherein the relatively high solubility of polar lipid in an aqueous solution of the water-soluble organic solvent, in which the aqueous solution comprises from 80 to 95 percent by weight water-soluble organic solvent followed by employing the use of a water-soluble organic solvent, wherein the relatively high solubility of polar lipid in an aqueous solution of the water-soluble organic solvent, in which aqueous solution comprises from 25 to 30 percent by weight water-soluble organic solvent, are employed to assist in said recovery.

17. The process of any of Claims 1-16, wherein said polar lipid comprises a phospholipid.

18. The process of any of Claims 1-17, wherein at least a portion of said process is performed in an oxygen-reduced atmosphere.

19. The process as claimed in Claim 1, wherein said steps of adding and subjecting are repeated at least once.

## Patentansprüche

1. Verfahren zur Fraktionierung eines Öl, polares Lipid und Protein enthaltenden Gemischs, das aus Mikroben erhalten wurde, folgende Schritte umfassend:
a) Zugabe eines wasserlöslichen organischen Lösemittels zu dem Gemisch und Trennung des Proteins von dem Gemisch zur Bildung einer proteinreichen Fraktion und einer an polarem Lipid/Öl reichen Fraktion;
b) Reduzieren der Konzentration des wasserlöslichen organischen Lösemittels in der an polarem Lipid/Öl reichen Fraktion, und
c) Unterziehen des Wassers/wasserlöslichen organischen Lösemittels und der an polarem Lipid/Öl reichen Fraktion einer Dichtetrennung zur Bildung einer an polarem Lipid reichen Fraktion und einer ölreichen Fraktion;
d) wobei die in Schritt (a) gebildete an polarem Lipid/Öl reiche Fraktion von 5 bis 40 Gew.-% polares Lipid und von 60 bis 95 Gew.-% Öl umfasst, und
e) wobei die in Schritt (a) gebildete proteinreiche Fraktion von 80 bis 95 Gew.-% Protein auf Trockenbasis umfasst; und
f) wobei das wasserlösliche organische Lösemittel in Schritt (a) in einem Gemisch aus wasserlöslichem organischen Lösemittel und Wasser vorliegt, wobei das Gemisch aus wasserlöslichem organischen Lösemittel und Wasser mindestens 68 Gew.-% wasserlösliches organisches Lösemittel umfasst;
g) wobei das wasserlösliche organische Lösemittel in Schritt (c) in einem Gemisch aus wasserlöslichem organischen Lösemittel und Wasser vorliegt, wobei das Gemisch aus wasserlöslichem organischen Lösemittel und Wasser von 25 bis 30 Gew.-% wasserlösliches organisches Lösemittel umfasst.

2. Verfahren gemäß Anspruch 1, wobei die Trennung von Protein von Schritt (a) folgende Schritte umfasst:
(a) Zugabe eines wasserlöslichen organischen Lösemittels zu dem Öl, polares Lipid und Protein enthaltenden Gemisch zur Erzielung einer Konzentration des wasserlöslichen organischen Lösemittels von mindestens 80 Gew.-%, und
(b) Trennen des resultierenden Gemischs durch Dichtetrennung in eine proteinreiche Fraktion und eine an polarem Lipid/Öl reiche Fraktion.

3. Verfahren gemäß Anspruch 1, wobei das wasserlösliche organische Lösemittel nach der Dichtetrennung aus der proteinreichen Fraktion und der an polarem Lipid/Öl reichen Fraktion zurückgewonnen wird.

4. Verfahren gemäß Anspruch 1, wobei das Öl, polares Lipid und Protein enthaltende Gemisch ferner Cholesterin umfasst und eine erhebliche Menge des Cholesterins auf die ölreiche Fraktion im Anschluss an die Trennung von Schritt (c) verweist.

5. Verfahren gemäß Anspruch 1, wobei das wasserlösliche organische Lösemittel in Schritt (a) in einem Gemisch aus wasserlöslichem organischen Lösemittel und Wasser vorliegt, wobei das Gemisch aus wasserlöslichem organischen Lösemittel und Wasser von 80 bis 95 Gew.-% wasserlösliches organisches Lösemittel enthält.

6. Verfahren gemäß Anspruch 1, wobei das wasserlösliche organische Lösemittel durch Gegenstromwäsche, Verdampfen oder Trocknen gewonnen wird.

7. Verfahren gemäß Anspruch 1, wobei die Polarlipid-reiche Fraktion getrocknet wird, um wasserlösliches organisches Lösemittel zurückzugewinnen, mit einem Gemisch aus einem wasserlöslichen organischen Lösemittel und Wasser gewaschen wird, das mehr als 80 Gew.-% wasserlösliches organisches Lösemittel umfasst, um das restliche Protein auszufällen, und ferner getrocknet wird, um das wasserlösliche organische Lösemittel zurückzugewinnen.

8. Verfahren gemäß Anspruch 7, wobei die Zugabe des wasserlöslichen organischen Lösemittels die Ausfällung von mindestens einem Teil des Proteins bewirkt, das durch Dichtetrennung gewonnen wird.

9. Verfahren gemäß Anspruch 1 - 8, wobei das wasserlösliche organische Lösemittel ein polares Lösemittel umfasst.

10. Verfahren gemäß Anspruch 1 - 9, wobei das wasserlösliche organische Lösemittel einen Alkohol umfasst.

11. Verfahren gemäß Anspruch 1 - 10, wobei das wasserlösliche organische Lösemittel einen C₁-C₈-Alkohol umfasst.

12. Verfahren gemäß Anspruch 1 - 11, wobei das wasserlösliche organische Lösemittel Isopropanol, Ethanol oder Gemische davon umfasst.

13. Verfahren gemäß Anspruch 1 - 12, wobei der pH-Wert während der Verarbeitung zwischen pH 4 und pH 10 liegt.

14. Verfahren gemäß Anspruch 1 - 13, wobei mindestens 60% der ursprünglich in dem Gemisch vorhandenen polaren Lipide in einer an polarem Lipid reichen Fraktion gewonnen werden.

15. Verfahren gemäß Anspruch 1 - 14, wobei mindestens 80% der ursprünglich in dem Gemisch vorhandenen polaren Lipide in einer an polarem Lipid reichen Fraktion gewonnen werden.

16. Verfahren zur Gewinnung von polarem Lipid aus einem polares Lipid enthaltenden Gemisch, das aus Mikroben erhalten wurde, in dem ein wasserlösliches organisches Lösemittel benutzt wird, worin die relativ hohe Löslichkeit von polarem Lipid in einer wässrigen Lösung des wasserlöslichen organischen Lösemittels, in dem die wässrige Lösung von 80 bis 95 Gew.-% wasserlösliches organisches Lösemittel umfasst, gefolgt von der Nutzung eines wasserlöslichen organischen Lösemittels, worin die relativ hohe Löslichkeit von polarem Liquid in einer wässrigen Lösung des wasserlöslichen organischen Lösemittels, in dem die wässrige Lösung von 25 bis 30 Gew.-% wasserlösliches organisches Lösemittel umfasst, dazu benutzt werden, den Gewinnungsprozess zu unterstützen.

17. Verfahren gemäß einem der Ansprüche 1 - 16, worin das polare Lipid ein Phospholipid umfasst.

18. Verfahren gemäß einem der Ansprüche 1 - 17, wobei mindestens ein Teil des Verfahrens in einer sauerstoffreduzierten Atmosphäre durchgeführt wird.

19. Verfahren gemäß Anspruch 1, wobei die Schritte der Zugabe und des Unterziehens mindestens einmal wiederholt werden.

## Revendications

1. Procédé pour fractionner un mélange contenant de l'huile, des lipides polaires et des protéines obtenu à partir de microbes, comprenant les étapes consistant à :
a) ajouter un solvant organique soluble dans l'eau audit mélange et séparer les protéines dudit mélange, afin de former une fraction riche en protéines et une fraction riche en huile/lipides polaires ;
b) réduire la concentration de solvant organique soluble dans l'eau dans ladite fraction riche en huile/lipide polaires et
c) soumettre le solvant organique soluble dans l'eau/l'eau et la fraction riche en huile/lipides polaires à une séparation par densité afin de former une fraction riche en lipide polaire et une fraction riche en huile,
d) ladite fraction riche en lipide polaire/huile formée dans l'étape (a) comprenant de 5 % à 40 % en poids de lipide polaire et de 60 % à 95 % en poids d'huile, et
e) ladite fraction riche en protéine formée dans l'étape (a) comprenant de 80 % à 95 % en poids de protéine sur une base sèche, et
f) ledit solvant organique soluble dans l'eau dans l'étape (a) étant présent dans un mélange solvant organique soluble dans l'eau/eau, dans lequel ledit mélange solvant organique soluble dans l'eau/eau comprend au moins 68 % en poids de solvant organique soluble dans l'eau ;
g) dans lequel ledit solvant organique soluble dans l'eau de l'étape (c) est présent dans un mélange solvant organique soluble dans l'eau/eau, dans lequel ledit mélange solvant organique soluble dans l'eau/eau comprend de 25 % à 30 % en poids de solvant organique soluble dans l'eau.

2. Procédé selon la revendication 1, dans lequel la séparation des protéines dans l'étape (a) comprend les étapes consistant à :
(a) ajouter un solvant organique soluble dans l'eau audit mélange contenant de l'huile, un lipide polaire et des protéines, pour obtenir une concentration de solvant organique soluble dans l'eau d'au moins 80 % en poids ; et
(b) séparer par densité le mélange obtenu en une fraction riche en protéine et une fraction riche en huile/lipides polaires.

3. Procédé selon la revendication 1, dans lequel le solvant organique soluble dans l'eau est récupéré dans la fraction riche en protéines et la fraction riche en huile/lipides polaires après la séparation par densité.

4. Procédé selon la revendication 1, dans lequel ledit mélange contenant de l'huile, des lipides polaires et des protéines comprend en outre du cholestérol et une quantité sensible dudit cholestérol se rapporte à ladite fraction riche en huile conformément à la séparation de l'étape (c).

5. Procédé selon la revendication 1, dans lequel ledit solvant organique soluble dans l'eau dans l'étape (a) est présent dans un mélange solvant organique soluble dans l'eau/eau, dans lequel ledit mélange solvant organique soluble dans l'eau/eau comprend de 80 % à 95 % en poids de solvant organique soluble dans l'eau.

6. Procédé selon la revendication 1, dans lequel ledit solvant organique soluble dans l'eau est récupéré par un lavage à contre-courant, évaporation ou séchage.

7. Procédé selon la revendication 1, dans lequel ladite fraction riche en lipides polaires est séchée pour récupérer le solvant organique soluble dans l'eau, lavée avec un mélange solvant organique soluble dans l'eau/eau, comprenant plus de 80 % en poids de solvant organique soluble dans l'eau, afin de précipiter la protéine résiduelle et ultérieurement séchée pour récupérer le solvant organique soluble dans l'eau.

8. Procédé selon la revendication 7, dans lequel l'addition dudit solvant organique soluble dans l'eau provoque la précipitation d'au moins une partie de ladite protéine, qui est récupérée par séparation par densité.

9. Procédé selon les revendications 1-8, dans lequel ledit solvant organique soluble dans l'eau comprend un solvant polaire.

10. Procédé selon les revendications 1-9, dans lequel ledit solvant organique soluble dans l'eau comprend un alcool.

11. Procédé selon les revendications 1-10, dans lequel ledit solvant organique soluble dans l'eau comprend un alcool en C₁-C₈.

12. Procédé selon les revendications 1-11 dans lequel ledit solvant organique soluble dans l'eau comprend de l'isopropanol, de l'éthanol ou des mélanges de ceux-ci.

13. Procédé selon les revendications 1-12, dans lequel le pH pendant le traitement est de 4 à 10.

14. Procédé selon les revendications 1-13, dans lequel au moins 60 % des lipides polaires présents à l'origine dans le mélange sont récupérés dans une fraction riche en lipides polaires.

15. Procédé selon les revendications 1-14, dans lequel au moins 80 % des lipides polaires présents à l'origine dans le mélange sont récupérés dans une fraction riche en lipides polaires.

16. Procédé de récupération de lipides polaires d'un mélange contenant des lipides polaires obtenus auprès de microbes, en utilisant un solvant organique soluble dans l'eau, ayant une solubilité relativement élevée du lipide polaire dans une solution aqueuse du solvant organique soluble dans l'eau, dans lequel la solution aqueuse comprend de 80 à 95 pour cent en poids de solvant organique soluble dans l'eau, puis en utilisant un solvant organique soluble dans l'eau, ayant une solubilité relativement élevée de lipide polaire dans une solution aqueuse du solvant organique soluble dans l'eau, dans lequel la solution aqueuse comprend 25 à 30 pour cent en poids de solvant organique soluble dans l'eau, pour aider à ladite récupération.

17. Procédé selon l'une quelconque des revendications 1-16, dans lequel ledit lipide polaire comprend un phospholipide.

18. Procédé selon l'une quelconque des revendications 1-17, dans lequel au moins une partie dudit procédé est effectuée dans une atmosphère réduite en oxygène.

19. Procédé selon la revendication 1, dans lequel lesdites étapes d'addition et de soumission sont répétées au moins une fois.
